Europäisches Patentamt

European Patent Office

Office européen des brevets

(11) Publication number: **0 074 426**

**B1**

# (12) EUROPEAN PATENT SPECIFICATION

(45) Date of publication of patent specification: **21.11.85**

(21) Application number: **81107236.2**

(22) Date of filing: **14.09.81**

(51) Int. Cl.⁴: **A 61 K 9/28,** A 61 K 9/52, A 61 K 33/10

(54) Pharmaceutical oral dosage forms of an active agent capable of forming or releasing bicarbonate ions.

(43) Date of publication of application:
**23.03.83 Bulletin 83/12**

(45) Publication of the grant of the patent:
**21.11.85 Bulletin 85/47**

(84) Designated Contracting States:
**AT BE CH DE FR GB IT LI NL SE**

(56) References cited:
FR-A-1 272 923
GB-A-1 270 781

CHEMICAL ABSTRACTS, vol. 92, no. 18, May 1980, page 412, col. 1, no. 153203t, Columbus Ohio (USA);
CHEMICAL ABSTRACTS, vol. 81, no. 10, 9th September 1974, page 324, col. 1, no. 54466x, Columbus Ohio (USA);
CHEMICAL ABSTRACTS, vol. 87, no. 26, 26th December 1977, page 291, columns 1-2, no. 206530c, Columbus Ohio (USA);

(73) Proprietor: **Olima Holding AG**
**Bauernhofstrasse 14**
**CH-8853 Lachen, Schwyz (CH)**

(72) Inventor: **Helbig, Joachim, Dr. med. vet.**
**Traubinger Strasse 23**
**D-8231 Tutzing (DE)**
Inventor: **Kopp, Klaus F. Dr.**
**Aschlkofener Strasse 4**
**D-8019 Ebersberg (DE)**

(74) Representative: **Patentanwälte Müller-Boré,**
**Deufel, Schön, Hertel, Lewald, Otto**
**Postfach 26 02 47 Isartorplatz 6**
**D-8000 München 26 (DE)**

(56) References cited:
THE MERCK INDEX, 8th edition, 1968, Merck & Co., page 954, right-hand column, Rahway, New York (USA); "Sodium bicarbonate"
HAGERS HANDBUCH DER PHARMAZEUTISCHEN PRAXIS, 4th edition, vol. 6, 1977, pt. A: N-Q, pages 94-96, Springer Verlag, Berlin (DE);

Courier Press, Leamington Spa, England.

## Description

This invention relates to novel agents and oral dosage forms of an active substance for treating patients suffering from abnormal distribution and retention of body fluids or retention products. Accompanied by corrections of body fluid distributions, such as may be achieved by increasing renal function with oral dosage forms of the invention, increased passage of retention products normally entrained in the urine can also be achieved. Furthermore, oral dosage forms of the invention are useful for prophylactic treatment of patients liable to contract renal failure. The invention also enables prophylactic treatment and treatment of patients having or being inclined to form kidney stones. In general, the common feature of the variety of ailments which can be treated or the corrective measures which can be taken are associated with altered renal function or renal insufficiency.

Previous attempts which might be said to relate to the considerations of the present invention have not comprised recognition of the patient condition which should be established in order to achieve the treatment procedures indicated, and in any event no convenient means for establishing such patient condition have been available.

Physiological studies have shown that a deficit of bicarbonate in the blood and extracellular fluid can lead to a condition described as "acidotic volume expansion".

Another physiological phenomenon is that the healthy kidney generates and retains more bicarbonate in the body when a deficiency of fluid in the blood and extracellular volume arises. In this fashion, a healthy patient subjected to body fluid volume contraction will enter into a state of moderate alkalosis.

The above physiological phenomena have not in the past been recognised as providing potential for therapeutic treatment of ailments and illnesses associated with altered kidney function. Thus, although there have been suggestions in the past to attempt neutralising chronic states of metabolic acidosis, for example by intravenous injection of sodium bicarbonate solution, such procedures have in general been considered to involve some danger to the patient, particularly when the patient is suffering from high blood pressure, as is frequently the case. Similarly, the generally recognised teachings suggest that hypersodemia can develop following on such administration which can lead to such consequences as high blood pressure, cardiac insufficiency, and pulmonary and peripheral edema. Overall, there is a strong prejudice against administration of sodium bicarbonate in the treatment of ailments associated with altered renal function. What is not generally known however is that complications such as have been experienced and described, are in fact not as a direct result of the presence of sodium bicarbonate itself, but rather of secondary changes in sodium, potassium, and calcium levels, this could not easily be recognised in view of the lack of availability of suitable laboratory methods.

Attempt to neutralise, even in part, metabolic acidosis for example by administration of sodium bicarbonate has in general been avoided for reasons such as already mentioned and more particularly since bicarbonate loading of a patient has incorrectly been considered to depress renal function.

In contrast to practically all indications of the past, it has been found in accordance with the invention that administration of an alkaline acting substance, in particular one which forms or releases bicarbonate ions, in sufficient amount to raise patient plasma bicarbonate level, can in a large number of cases be highly effectively employed in the therapeutic or prophylactic treatment of a large variety of ailments involving abnormal distribution and retention of body fluids such as may be the result of altered renal function.

Furthermore, it has been found that acute renal failure can be prevented by prophylactic oral administration of quantities of a bicarbonate provided in a special pharmaceutical oral dosage form of the invention, for example preliminary to severe medical and surgical treatment, or immediately following severe intoxications. Exemplary are kidney transplant recipients and donors, and preoperative conditioning of patients with varying degrees of renal insufficiency, such as may be related to diabetes, age and pregnancy.

In connection with establishing doses to be administered, it is most preferable to establish a so-called "plasma base excess". A consideration related to this preference is the fact that observations made in reversing of acute renal failure, namely to the point where polyuria sets in, have reflected a preliminary almost simultaneous increase in fresh patient urine pH value to about neutral or alkaline suggesting passage of excess bicarbonate.

It is recognised that patients suffering from chronic renal disease characteristically display, irrespective of the type of such disease, and increase in retention products, such as hydrogen ions, sodium, urea, creatinine and uric acid, which are normally discharged through the kidneys. When these substances attain a certain level, a clinical picture of uremia is obtained, which is a threat to life and can ultimately only be treated with the aid of the artificial kidney. Due to the increased concentration of hydrogen ions, which accompanies the increase of substances which should be eliminated by urine entrainment, hyperacidity or, metabolic acidosis results. Hence, patients with advanced renal insufficiency characteristically display a marked hyperacidity, which has also been recognised to be at least a partial cause of various other states of ill health, such as disorders in bone metabolism and mineral metabolism.

From the experience in treating, preventing and conditioning against acute renal failure, the possibilities of advantage of neutralising at least in part metabolic acidosis as described above have in spite of countless earlier attempts been further examined. Administration of substances having an alkaline effect in an effort to neutralise metabolic acidosis have, for example, been attempted by intravenous injection of sodium bicarbonate solutions;

K. E. Thoma in "Med. Klin." *71* (1976) 124—127. Furthermore, the oral administration of a hexa potassium - hexasodium - penta - citrate - hydrate - complex or a potassium-free hexacalcium - hexasodium - heptacitrate - hydrate - complex is described in "Diagnostik" *10* (1977) 569—572. However, intravenous injection would not be an acceptable procedure for therapy of chronic renal insufficiency and the oral administration described leaves much to be desired in that larger amounts of these complexes need to be taken with large amounts of liquid, which frequently causes flatulence, nausea, diarrhoea and vomiting. Moreover, these complexes are in part eliminated undigested. It is accordingly difficult or impossible to achieve adequate bicarbonate levels produced by metabolism of the complexes for correcting metabolic acidosis.

Also generally known for many years is the use of sodium bicarbonate for treating acid indigestion, again procedure creating flatulence, nausea and possibly vomiting. Such administration basically involves treatment of hyperacidity in the stomach and generally involves single irregular doses. Attempt which might be made to employ sodium bicarbonate to neutralise at least in part metabolic acidosis can only have limited success in that relatively large amounts would be required to raise plasma bicarbonate content sufficiently to exercise an adequate corrective effect on metabolic acidosis. It is noted here that the amounts of sodium bicarbonate required for correcting metabolic acidosis would lead to development of substantial amounts of sodium chloride in the stomach, an increased discharge of hydrochloric acid by the stomach and hence yet further development of amounts of sodium chloride. Patients suffering from renal insufficiency are inclined to suffer from hypertension and increased amounts of sodium chloride, which has a hypertensive effect, is accordingly of strong disadvantage particularly in that hypertension is of high danger to the patient's life expectancy.

The overall views held today in the medical profession is that attempt to neutralise metabolic acidosis in chronic renal failure, leads to disadvantages which outweigh any advantages which may be achieved. A variety of reasons are reported, and there exists an overall prejudice against administration of sodium bicarbonate. One example additional to hypertension is that increased sodium in the body leads to edema.

It is an object of this invention, to establish an acceptable procedure for raising plasma bicarbonate levels without need to resort to intravenous administration (such as would be necessary in acute patient conditions) and avoiding complications of the nature discussed above. Essentially, it has been found that increase of plasma bicarbonate levels is of extreme benefit in spite of the prejudice against such approach. Indeed, the finding of an acceptable procedure for increasing plasma bicarbonate levels has revealed extraordinary utility in the most varying types of ailments, including practically any form of abnormal body fluid retention. For example, elderly patients (who might also be suffering from incomplete renal function) exhibiting such conditions as dropsy, peripheral edemas, varicose veins and like disturbances have in many cases responded extraordinarily well to treatment with pharmaceutical oral dosage forms of the present invention.

Particularly, the present studies and the pharmaceutical preparation which has emanated therefrom, is related to treatment of abnormal distribution and retention of body fluids which result from altered renal function.

It has been found that increase of plasma bicarbonate levels to correct at least in part metabolic acidosis existent in patients suffering from renal insufficiency has extraordinarily and surprisingly beneficial effects. Thus, it has been found that substantial correction of metabolic acidosis in such patients can in a large number of cases enormously increase urine production and similarly enormously increase clearance of retention products. Effects are in some cases so marked that patients who would in due course need to be subjected to blood dialysis can either avoid being subjected to this form of treatment at all, or such treatment can be considerably postponed. Apparently, the increase in plasma bicarbonate level and consequent correction of metabolic acidosis induces an alkalotic volume contraction of the nature discussed in the initial stages of this specification. This driving force is believed to enable remaining functioning sections of the kidney to increase in function, i.e. urine output, sometimes to an extent sufficient for a patient, even dialysis patients to remain in an adequate metabolic state of water and acid-base balance. Moreover, the state of bicarbonate-induced alkalotic volume contraction may have the effect of lowering elevated blood pressure, which is a finding of considerable surprise since all indications to date are that sodium bicarbonate administration leads to the reverse result.

The procedure for treatment of the above nature involves increasing plasma bicarbonate levels by oral administration of oral dosage forms of the present invention.

More particularly, although other alkaline acting substances might find application in this method of the present invention, the alkaline acting substance would be adapted to form or release bicarbonate ions. Release of bicarbonate ions and availability thereof to restore depleted extra-cellular reserves in metabolic acidosis is at this time considered to be a key factor.

It is most preferable to administer sufficient alkaline acting substance to achieve a plasma base excess in the patient, which can for example very easily be monitored by determining patient fresh urine pH value. Thus, for example, patient fresh urine pH values in the neutral to alkaline region provides a convenient means to confirm a condition of moderate alkalosis.

The means to establish the high alkaline level by oral administration necessary to achieve increased plasma bicarbonate level is achieved in a fashion considered to be of great importance and value in that it involves both the entirely different therapeutic approach described above and also a special pharmaceutical oral dosage form, never previously available in a satisfactory form.

The alkaline acting substance comprised in the oral dosage form of the present invention is easily resorbed and very surprisingly this takes place without the variety of complications such as mentioned in conjunction with administration of alkaline acting substances employed in the past. More particularly, the alkaline acting substance, which is one capable of forming or releasing bicarbonate ions, is formed into a pharmaceutical oral dosage form adapted to release the alkaline acting substance only in the alkaline environment of the small intestine, notably at resorption sites. Very considerably higher rates of resorption as compared to resorption which may take place following on a release of the alkaline acting substance in the acid environment of the stomach can be achieved. Accordingly, correspondingly lower dosages to achieve the desired elevated plasma bicarbonate levels may be employed.

In accordance with the invention, there is provided a pharmaceutical oral dosage form comprising as active agent a bicarbonate as a bicarbonate ion forming substance, the active agent being enclosed in enclosure means such that the active agent is exposed only in the intestine of a subject to form or release resorbable bicarbonate ions without the possibility of reaction thereof with gastric secretions in the stomach of the subject. The bicarbonate ion forming substance may surprisingly comprise sodium bicarbonate and may be associated with an amount of a carbonate such as sodium carbonate.

The terms exposed "only in the intestine" and "resorbable" as employed herein are intended to provide distinction over known pharmaceutical oral dosage forms such as may comprise a bicarbonate which are either completely or partially destroyed by the presence of an acid or by the formation of at least some reaction products which are the cause of reported side effects of the nature discussed herein. The pharmaceutical oral dosage forms of the invention are characterised by being substantially free of undesirable side effects known to exist for oral dosage forms of gastrointestinal bicarbonate-releasing preparations. The active agent employed in the dosage forms of the invention essentially needs to be one which forms or releases or which has been adapted in the oral dosage form to form or release bicarbonate ions only in the intestine of a subject, without the possibility of reaction thereof (or any significant part thereof which leads to said side effects) with gastric secretions in the stomach of the subject.

The active agent may be associated with an additional active agent also suitable for the prophylaxis and therapy of kidney stones, selected from the group consisting of an alkali metal or alkaline earth metal salt of citric acid, and urea. It is however to be appreciated that, and as is mentioned above, the pharmaceutical oral dosage form of the invention is on its own, i.e. without further active agents, useful in the prophylactic treatment and treatment of patients having or being inclined to form kidney stones.

In the pharmaceutical oral dosage form of the invention, the active agent may be associated with a calcium compound, such as calcium gluconate, suitable for supplementing calcium in a subject. Similarly, the active agent may be associated with a potassium compound, such as one selected from the group consisting of potassium chloride, potassium bicarbonate, and potassium citrate, suitable for supplementing potassium in the subject. Furthermore, trace elements recognised to contribute to the general well-being of subjects may optionally be comprised in the dosage form of the invention.

Aspartic acid or a derivative thereof, which also encourages increase of urine volume, may additionally be included in the oral dosage form of the invention. Magnesium aspartate or magnesium aspartate hydrochloride may be the aspartic acid derivative, which then also provides means for supplementing magnesium in a subject.

The enclosure means enclosing the active agent so that it is exposed only in the intestine of a subject to form or release resorbable bicarbonate ions without the possibility of reaction thereof with gastric secretions in the stomach of the subject is of high importance in the present invention, since if this not entirely or sufficiently effective to achieve exposure of the active agent only in the intestine, i.e. at resorption sites, the active agent may react with gastric secretions and hence lead to formation of substances, notably sodium chloride, which are undesirable and which lead to the undesirable side-effects reported for all known sodium bicarbonate oral dosage forms. Provided that this is effective, however, the enclosure means may in general comprise a gastric-secretion-resistant intestine-secretion-decomposable material. The enclosure means may for example comprise an acrylic resin. Various enclosure means may be divised and include such enclosures as a gelatine capsule enclosure rendered resistant to gastric secretions, for example with the aid of hydroxypropyl methyl cellulose phthalate and dibutyl phthalate, or a tablet enclosure which is rendered resistant to gastric secretions, for example with the aid of cellulose acetate phthalate.

Although, gastric-secretion-resistant intestine-secretion-decomposable powder and granulate forms of the pharmaceutical oral dosage form are not excluded, and indeed granulate forms of the active agent are specifically contemplated by the present invention, it is preferred to provide unit oral dosage forms, comprising from about 200 mg to about 1500 mg of the bicarbonate, in particular sodium bicarbonate.

The present invention extends to the use of rather the indication that the pharmaceutical oral dosage forms of the invention are useful as an agent for increasing the plasma bicarbonate level of a mammalian subject and which also enables prophylactic or therapeutic effects to be achieved in the subject (without side effects for example reported for known sodium bicarbonate oral dosage forms) by virtue of the entirely or sufficiently effective enclosure means discussed above. The prophylactic effects which may be achieved more specifically include prevention of acute renal failure in a subject liable to contract renal failure, for example preliminary to severe medical and surgical treatment, or immediately following severe intoxications. Another prophylactic effect more specifically includes prevention of the formation of kidney stones. The therapeutic effects which may be achieved more specifically include treatment of a mammalian subject suffering from abnormal retention of body fluids or retention products, for example as is the case in a condition of metabolic acidosis, where the subject is suffering from altered renal function or renal insufficiency, and various other ailments such as fluid overload which are in general accompanied by this condition. Another more specific therapeutic effect which may be achieved is the treatment of a subject suffering from kidney stones.

A container comprising a plurality of unit dosage forms of the invention, may in accordance with the invention be accompanied by indications of suitability of its contents for use in the prophylaxis or therapy of abnormal distribution or retention of body fluids or retention products in a mammalian subject, more specifically mentioned above.

An additional factor which is an important contribution related to the present invention is the amount or dose specified in instructions accompanying the container. These instructions, in accordance with the invention, may indicate that the specified amount is sufficient to establish fresh urine pH value of the subject in the neutral to alkaline range at a pH of at least 6.5 and preferably at a pH of between 6.8 and 8. Furthermore, the specified amount may be indicated in the instructions to be sufficient to maintain said fresh urine pH value.

It has also been found that oral dosage forms of the invention, i.e. oral dosage forms adapted to form or release resorbable bicarbonate ions only in the intestine of a subject, may comprise a carbonate, such as sodium carbonate. Thus, provided that regions of over-alkalinity which can lead to irritation or damage to the walls of the small intestine can be avoided, carbonate ion release in the small intestine of a subject may lead to formation of two bicarbonate ions via reaction with water and reaction of liberated hydroxy ions with carbon dioxide available.

Exemplary unit oral dosage forms of the invention may comprise the following constituents;

Core compositions
1) Bicarbonate forming
   or releasing substance        200 to 1000 mg

2) Adjuvants                     20 to 200 mg

3) Potassium supplement          0 to 100 mg

4) Magnesium supplement          0 to 500 mg

5) Calcium supplement            0 to 200 mg

6) Further active agents         0 to 200 mg

7) Trace element
   components                    0 to 10 mg

8) Colorants, carriers           0 to 100 mg

The core composition should possess a consistency enabling formation of a non-refractory tablet, dragee, granulate or capsule form. The form should also preferably be stable at temperatures well in excess of the temperature of warm-blooded animals, and preferably in excess of about 50°C. The formation of the dosage form should be effected in an environment of defined temperature and humidity conditions.

An exemplary coating composition suitable for coating a tablet form comprises the following essential constituents:
   Cellulose acetate phthalate
   Acrylic resin

An exemplary coating composition suitable for coating a capsule form to resist decomposition and reaction with gastric juices comprises the following essential constituents:
   Gelatine
   Hydroxymethylcellulose phthalate
   Dibutyl phthalate

Although it may be expected that pharmaceutical oral dosage forms as described above may be prepared with relative simplicity, practical experience has reflected that certain extreme care areas in the procedure need to be observed. Particularly, one area, when employing an alkaline acting agent and a coating which is sensitive to degradation at higher pH values, is that very substantially anhydrous conditions need to be observed throughout the preparation procedure. Another area is that the active agent needs to be uniformly and completely enclosed within a coating of pH-sensitive substance, without any points of weakness enabling premature penetration of the coating.

In accordance with the invention, there is furthermore provided a process for producing a pharmaceutical oral dosage form of an active agent capable of forming or releasing bicarbonate ions, which comprises the steps of preparing a solution of a pH-sensitive substance which is decomposable at a pH in excess of about 6, said substance being very substantially free of water and the solvent employed for preparing said solution similarly being very substantially free of water, preparing a core of said active agent which is also very substantially free of water, creating an atmosphere about said core which has a relative humidity of lower than 40%, applying said solution of the pH-sensitive substance to the surface of said core, and allowing said solvent to evaporate to obtain a pharmaceutical oral dosage form of the active agent which is uniformly and completely enclosed within a coating of said pH-sensitive substance.

The pH-sensitive substance may be celluloseacetate-phthalate and the solvent employed for preparing a solution of the pH sensitive substance would be of a non-alcoholic hydrocarbon having from 1 to 5 carbon atoms, possessing ready volatility. A halogenated hydrocarbon such as methylene chloride is preferred. The solution of the pH-sensitive substance may comprise a softening agent, pigment or protective agent. The protective agent would preferably impart a gloss to the surface of a final pharmaceutical oral dosage form.

An additional important factor in preparing pharmaceutical oral dosage forms of the invention is that the coating procedure proceed at high rate. This may be achieved by including a relatively high proportion by weight of the pH-sensitive substance in the solution, such as in excess of 5% by weight, and preferably between 7 to 10% by weight. Furthermore, in order to achieve said high rate coating, the above mentioned readily volatile solvent is employed and the atmosphere about the core to which the solution is to be applied is maintained at a relatively high temperature, in excess of 35°C and preferably higher than 40°C but lower than 55°C.

In order to ensure that a uniform and completely enclosed dosage form is achieved, the core is freed of all sharp corners and irregularities at the surface to obtain a polished surface core form which is very substantially free of water. The core of the active agent may be produced by initially forming a granulate which is very substantially free of water and pressing the granulate to obtain a core form comprising a unit dosage amount of the active agent.

The granulate, additional to comprising the active agent would in general comprise a granulating agent dissolved in a solvent, and optionally a binding agent, each component being very substantially free of water, and in which said granulate is freed of solvent before being pressed to obtain the core form. The granulate may be associated with an expanding agent to encourage distribution of the active agent when this is set free in the intestine of a mammalian subject.

To ensure that no particles, particularly of active agent adhere to coatings applied in the procedure described above, the atmosphere surrounding the polished surface core form is entirely freed of all core form components before applying said solution of the pH-sensitive substance to the surface of said core.

A specific example of a core composition and unit dosage form is as follows:

| | |
|---|---|
| Sodium bicarbonate | 750.000 mg |
| Polyvinylpyrrolidone | 22.500 mg |
| Binding agent (glycerides) | 20.000 mg |
| Potato starch | 10.000 mg |
| Silicium dioxide, colloidal | 5.000 mg |
| Magnesium stearate | 15.000 mg |
| | 822.500 mg |

A specific example of a coating composition, in an amount for a unit dosage form is as follows:

| | |
|---|---|
| Celluloseacetate phthalate | 64.000 mg |
| Softening agent | 7.300 mg |
| Pigments | 6.800 mg |
| Protecting agent (gloss) | 2.800 mg |
| | 80.900 mg |

Exemplary unit dosage forms found to be convenient comprise from about 200 to about 1500 mg of alkaline acting substance, such as sodium bicarbonate. Unit dosage forms may be tablets, capsules or dragees enclosed by an acid-resistant (gastric-juice resistant) alkali-decomposable enclosure.

It is appropriate to mention at this juncture, and particularly in relation to dosages to be administered in accordance with treatment procedures and in accordance with the present invention, that correction of metabolic acidosis insofar as such may have been attempted in the past, has lacked proper recognition of the necessary plasma bicarbonate level, the need for maintenance of such level, and at least in the case of treatment of metabolic acidosis conditions (of even slight degree) which are existent in renal insufficiency circumstances, means whereby sufficient alkaline acting substance such as a substance capable of releasing a cation such as sodium, and bicarbonate ions, has not been available.

Clinical evaluations have reflected that best results are achieved in the treatment of abnormal body fluid distribution such as result from altered renal function when the amount of alkaline acting substance administered is sufficient to create (and maintain) a moderate degree of metabolic alkalosis. Thus, as may be determined by blood gas analysis, bicarbonate concentrations in plasma should preferably lie above 24 mEq/l and most preferably above 26 mEq/l, which corresponds to about a 4 to 5 mEq/l base excess value.

Additional active agents, which may be included in the oral dosage forms of the invention are aspartic acid or derivatives thereof. Thus, it is observed that aspartic acid or derivatives thereof comprised in asparagus, increase urine production. An asparate specifically contemplated and which may be included in the oral dosage forms of the invention is magnesium aspartate or magnesium aspartate hydrochloride. This magnesium compound has also been found to lower uric acid values in patients receiving gout remedies.

Dosages which need to be applied to achieve values such as described above are of course dependent on the particular condition being treated. However, in general, it has been found that dosages ranging between 0.5 and 10 g/24 hours are adequate to establish preferred fresh patient urine pH value of above 6.5 and most preferably neutral to alkaline at a pH value between 6.8 and 8.

Indeed, most preferably, in view of indication reflected in the therapy of renal insufficiency, adequate renal function is regularly only established while fresh patient urine pH values are in the neutral to alkaline range.

It has already been indicated above that increased urine volume established by increased plasma bicarbonate levels leads to an associated increased elimination of retention products. Another surprising factor is that various conditions of edema, have been observed to be aleviated. This is of particular note in that increased sodium levels, such as would be expected from administration of sodium bicarbonate, have in the past been considered to be a cause of edema.

There has been mention above of treatment of a variety of ailments reflected by abnormal distribution and retention of body fluids, for example as may occur in old age, pregnancy and dietary indiscretion. The oral preparation of the present invention, it should be noted can advantageously comprise additional active agents. For example, the oral preparations of the present invention may comprise a nutrient such as glucose, phosphates, and amino acids, a diuretic, urea, digestive acids, and enzymes. Furthermore, for purposes of correcting possible states of hypocalcemia or potassemia an amount of a calcium product, such as calcium gluconate or an amount of a potassium compound, such as potassium chloride, potassium bicarbonate and potassium citrate, may be included in the oral preparations of the invention.

It has been observed above that the treatment procedures using the dosage form of the present invention may also comprise prophylactic approaches. One prophylactic approach worthy of mention is prevention of the formation of kidney stones. Such prophylactic action may be encouraged by inclusion in oral preparations of substances such as alkali or alkali earth metal citrates, or inclusion of urea itself. Thus, where increased urine volume may be encouraged by creating a state of alkalotic volume contraction, presence of increased amounts of citrate or urea in the urine volume can decrease tendencies of kidney stone formation. In like manner, presence of increased amounts of citrate or urea in increased urine volumes can regularly be successfully employed to assist in the elimination of kidney stone fines and kidney stones.

For purposes of maintaining a general state of well-being, particularly in elderly patients, trace elements today recognised to contribute to such may be included in oral preparations. Already mentioned is that elderly people commonly suffer from altered renal function.

A major consideration of the present invention is associated with the finding that effective therapeutical treatment of altered renal function can be achieved by oral administration of an alkaline acting substance, in particular one capable of liberating bicarbonate ions to replace bicarbonate reserves normally present in extracellular fluids, and more particularly sodium bicarbonate. Some consideration has been applied to methods which might be attempted to circumvent the concepts of this invention, for example by associating enteral administrations of pharmaceuticals of recognised therapeutic value with sodium bicarbonate. For this reason also, it is here explicitly stated that the present invention recognises and does not exclude oral preparations, particularly such as may be employed in the therapy of a large variety of diseases and ailments which can be as a result of partial or temporarily altered renal function, which comprises an alkaline acting substance such as sodium and bicarbonate ion liberating substances, in association with further pharmaceuticals.

Oral preparations of the present invention are highly effective in increasing urine volume when administered at correct minimum dosage levels as described and have been found to exercise extraordinarily beneficial effects in the therapy of a multitude of body fluid distribution and fluid retention disorders.

## Claims

1. A pharmaceutical oral dosage form comprising as active agent a bicarbonate as a bicarbonate ion forming substance, the active agent being enclosed in enclosure means such that the active agent is exposed only in the intestine of a subject to form a release resorbable bicarbonate ions without the possibility of reaction thereof with gastric secretions in the stomach of the subject.

2. A pharmaceutical oral dosage form according to claim 1, in which the bicarbonate ion forming substance comprises sodium bicarbonate.

3. A pharmaceutical oral dosage form according to claim 1 or claim 2, in which the bicarbonate is associated with an amount of a carbonate.

4. A pharmaceutical oral dosage form according to claim 3, in which the carbonate is sodium carbonate.

5. A pharmaceutical oral dosage form according to any one of the preceding claims, in which the active agent is associated with an additional active agent also suitable for the prophylaxis and therapy of kidney stones, selected from the group consisting of an alkali metal or alkaline earth metal salt of citric acid, and urea.

6. A pharmaceutical oral dosage form according to any one of the preceding claims, in which the active agent is associated with a calcium compound suitable for supplementing calcium in the subject.

7. A pharmaceutical oral dosage form according to claim 6, in which the calcium compound is calcium gluconate.

8. A pharmaceutical oral dosage form according to any one of the preceding claims, in which the active agent is associated with a potassium compound suitable for supplementing potassium in the subject.

9. A pharmaceutical oral dosage form according to claim 8, in which the potassium compound is selected from the group consisting of potassium chloride, potassium bicarbonate, and potassium citrate.

10. A pharmaceutical oral dosage form according to any one of the preceding claims, in which trace elements recognised to contribute to the general well-being of subjects are comprised in the oral dosage form.

11. A pharmaceutical oral dosage form according to any one of the preceding claims, in which aspartic acid or a derivative thereof is included in the oral dosage form.

12. A pharmaceutical oral dosage form according to claim 11, in which magnesium aspartate or magnesium aspartate hydrochloride is included in the oral dosage form.

13. A pharmaceutical oral dosage form according to any one of the preceding claims, in which the enclosure means comprises a gastric-secretion-resistant intestine-secretion decomposable material.

14. A pharmaceutical oral dosage form according to claim 13, in which the enclosure means comprises an acrylic resin.

15. A pharmaceutical oral dosage form according to claim 13, in which the enclosure means is a gelatine capsule enclosure rendered resistant to gastric secretions.

16. A pharmaceutical oral dosage form according to claim 13, in which the gelatine capsule enclosure is rendered resistant to gastric secretions with the aid of hydroxypropyl methyl cellulose phthalate and dibutyl phthalate.

17. A pharmaceutical oral dosage form according to claim 13 or claim 14, in which the gastric-secretion-resistant small-intestine-decomposable enclosure is a tablet enclosure rendered resistant to gastric secretions with the aid of cellulose acetate phthalate.

18. A pharmaceutical oral dosage form according to any one of the preceding claims, in unit dosage form, comprising from about 200 mg to about 1500 mg of the bicarbonate.

19. As an agent for increasing the plasma bicarbonate level of a mammalian subject and which also enables prophylactic or therapeutic effects to be achieved in the subject, a pharmaceutical oral dosage form according to any one of the preceding claims.

20. The agent of claim 19, in which the prophylactic effect to be achieved includes the prevention of acute renal failure in a subject liable to contract renal failure.

21. The agent of claim 19, in which the prophylactic effect to be achieved includes the prevention of the formation of kidney stones in a subject being inclined to form kidney stones.

22. The agent of claim 19, in which the therapeutic effect to be achieved is for treating a mammalian subject suffering from abnormal retention of body fluids or retention products.

23. The agent of claim 22, in which the treatment of abnormal distribution or retention of body fluids or retention products includes the treatment of a condition of metabolic acidosis.

24. The agent of claim 22, in which the treatment of abnormal distribution or retention of body fluids includes the treatment of a subject suffering from altered renal function or renal insufficiency.

25. The agent of claim 22, in which the treatment of abnormal distribution or retention of body fluids includes the treatment of a subject suffering from fluid overload.

26. The agent of claim 22, in which the treatment of abnormal distribution or retention of body fluids or retention products includes the treatment of a subject suffering from kidney stones.

27. A container comprising a plurality of unit dosage forms in accordance with claim 18, and being accompanied by indications of suitability of its contents for use in the prophylaxis or therapy of abnormal distribution or retention of body fluids or retention products in a mammalian subject.

28. A container comprising a plurality of unit dosage forms in accordance with claim 18, and being accompanied by instructions for a mammalian subject to swallow a specified amount of its contents for the prophylaxis or therapy of abnormal distribution or retention of body fluids or retention products in a mammalian subject.

29. The container of claim 28, in which the specified amount is indicated in the instructions to be sufficient to establish fresh urine pH value of the subject in the neutral to alkaline range at a pH of at least 6.5 and preferably at a pH of between 6.8 and 8.

30. The container of claim 29, in which the specified amount is indicated in the instructions to be sufficient to maintain said fresh urine pH value.

31. The container of any one of claims 27 to 30, in which the prophylaxis includes the prevention of actute renal failure in a patient liable to contract renal failure.

32. The container of any one of claims 27 to 30, in which the prophylaxis includes the prevention of the formation of kidney stones in a subject being inclined to form kidney stones.

33. The container of any one of claims 27 to 30, in which the therapy of abnormal distribution or retention of body fluids or retention products includes the treatment of a condition of metabolic acidosis.

34. The container of any one of claims 27 to 30, in which the therapy of abnormal distribution or retention of body fluids or retention products includes the treatment of a subject suffering from altered renal function or renal insufficiency.

35. The container of any one of claims 27 to 30, in which the therapy of abnormal distribution or retention of body fluids or retention products includes the treatment of a subject suffering from fluid overload.

36. The container of any one of claims 27 to 30, in which the therapy of abnormal distribution or retention of body fluids or retention products includes the treatment of a subject suffering from kidney stones.

37. A process for producing a pharmaceutical oral dosage form comprising as active agent a bicarbonate as a bicarbonate ion forming substance to form or release resorbable bicarbonate ions without the possibility of reaction thereof with gastric secretions in the stomach of the subject, which comprises the steps of preparing a solution of a pH-sensitive substance which is decomposable at a pH in excess of about 6, said substance being very substantially free of water and the solvent employed for preparing said solution similarly being very substantially free of water, preparing a core of said active agent which is also very substantially free of water, creating an atmosphere about said core which has a relative humidity lower than 40%, applying said solution of the pH-sensitive substance to the surface of said core, and allowing said solvent to evaporate to obtain a pharmaceutical oral dosage form of the active agent which is uniformly and completely enclosed within a coating of said pH-sensitive substance.

38. A process according to claim 37, in which the pH-sensitive substance is celluloseacetate-phthalate.

39. A process according to claim 37 or 38, in which the solvent employed for preparing the solution of the pH-sensitive substance is a non-alcoholic hydrocarbon having from 1 to 5 carbon atoms.

40. A process according to claim 39, in which the solvent is a halogenated hydrocarbon.

41. A process according to claim 40, in which the solvent is methylene chloride.

42. A process according to any one of claims 37 to 41, in which any one or more of a softening agent, pigment and protective agent are additionally comprised in the solution of the pH-sensitive substance.

43. A process according to any one of claims 37 to 42, in which the amount of pH-sensitive substance comprised in the solution is in excess of 5% by weight.

44. A process according to claim 43, in which the amount of pH-sensitive substance comprised in the solution is between about 7 and 10% by weight.

45. A process according to any one of claims 37 to 44, in which the coating of pH-sensitive substance is effected with relative rapidity by including a relatively high proportion by weight of the pH-sensitive substance in the solution, by employing readily volatile solvent and by maintaining the atmosphere about the core to which the solution is to be applied at a relatively high temperature, in excess of 35°C and preferably higher than 40°C but lower than 55°C.

46. A process according to any one of claims 37 to 45, in which the core of the active agent is produced by initially forming a granulate which is very substantially free of water, pressing the granulate to obtain a core form comprising a unit dosage amount of the active agent, and freeing the core of all sharp corners and irregularities at the surface to obtain a polished surface core form which is very substantially free of water.

47. A process according to claim 46, in which said granulate comprises the active agent, a granulating agent dissolved in a solvent, and optionally a binding agent, each component being very substantially free of water, and in which said granulate is freed of solvent before being pressed to obtain the core form.

48. A process according to claim 46 or claim 47, in which said granulate is associated with an expanding agent to encourage distribution of the active agent when this is set free in the intestine of a mammalian subject.

49. A process according to any one of claims 46 to 48, in which the atmosphere surrounding the polished surface core form is entirely freed of all core form components before applying said solution of the pH-sensitive substance to the surface of said core.

50. A process according to any one of claims 37 to 49, in which the bicarbonate ion forming substance comprises sodium bicarbonate.

51. A process according to any one of claims 37 to 50, in which the bicarbonate is associated with an amount of a carbonate.

52. A process according to claim 51, in which the carbonate is sodium carbonate.

53. A pharmaceutical oral dosage form comprising as active agent a bicarbonate as a bicarbonate ion forming substance, produced by the process of any one of claims 39 to 52.

## Patentansprüche

1. Pharmazeutische orale Dosierungsform, die als Wirkstoff ein Bikarbonat als Bikarbonationenliefernde Substanz aufweist, wobei der Wirkstoff derart in einer Umhüllung eingeschlossen ist, daß der Wirkstoff nur im Darm eines Patienten unter Bildung oder Freisetzung von resorbierbaren Bikarbonationen freigesetzt wird ohne die Möglichkeit einer Reaktion mit den Magensäften im Magen des Patienten.

2. Pharmazeutische orale Dosierungsform gemäß Anspruch 1, dadurch gekennzeichnet, daß die Bikarbonationenbildende Substanz aus Natriumbikarbonat besteht.

3. Pharmazeutische orale Dosierungsform gemäß Anspruch 1 oder 2, dadurch gekennzeichnet, daß das Bikarbonat mit einer Menge eines Karbonats vermischt ist.

4. Pharmazeutische orale Dosierungsform gemäß Anspruch 3, dadurch gekennzeichnet, daß das Karbonat aus Natriumkarbonat besteht.

5. Pharmazeutische orale Dosierungsform gemäß einem der vorhergehenden Ansprüche, dadurch gekennzeichnet, daß der Wirkstoff mit einem weiteren Wirkstoff vermischt ist, der ebenfalls für die Prophylaxe und Therapie von Nierensteinen geeignet ist, ausgewählt aus der Gruppe, die aus Alkalimetall- oder Erdalkalimetallsalzen von Zitronensäure sowie Harnstoff besteht.

6. Pharmazeutische orale Dosierungsform gemäß einem der vorhergehenden Ansprüche, dadurch gekennzeichnet, daß der Wirkstoff mit einer Kalziumverbindung vermischt ist, welche Kalzium in den Patienten zu ergänzen vermag.

7. Pharmazeutische orale Dosierungsform gemäß Anspruch 6, dadurch gekennzeichnet, daß die Kalziumverbindung aus Kalziumglukonat besteht.

8. Pharmazeutische orale Dosierungsform gemäß einem der vorhergehenden Ansprüche, dadurch gekennzeichnet, daß der Wirkstoff mit einer Kaliumverbindung, welche Kalium in dem Patienten zu ergänzen vermag, vermischt ist.

9. Pharmazeutische orale Dosierungsform gemäß Anspruch 8, dadurch gekennzeichnet, daß die Kaliumverbindung aus der Gruppe ausgewählt ist, die aus Kaliumchlorid, Kaliumbikarbonat und Kaliumzitrat besteht.

10. Pharmazeutische orale Dosierungsform gemäß einem der vorhergehenden Ansprüche, dadurch gekennzeichnet, daß Spurenelemente, welche für das allgemeine Wohlbefinden von Patienten anerkannt sind, in der oralen Dosierungsform enthalten sind.

11. Pharmazeutische orale Dosierungsform gemäß einem der vorhergehenden Ansprüche, dadurch gekennzeichnet, daß Asparaginsäure oder ein Derivat davon in der oralen Dosierungsform vorliegt.

12. Pharmazeutische orale Dosierungsform gemäß Anspruch 11, dadurch gekennzeichnet, daß Magnesiumaspartat oder Magnesiumaspartathydrochlorid in der oralen Dosierungsform enthalten ist.

13. Pharmazeutische orale Dosierungsform gemäß einem der vorhergehenden Ansprüche, dadurch gekennzeichnet, daß die Umhüllung aus einem Magensaft-resistenten und Darmsaftzersetzbaren Material besteht.

14. Pharmazeutische orale Dosierungsform gemäß Anspruch 13, dadurch gekennzeichnet, daß die Hülle aus einem Acrylharz besteht.

15. Pharmazeutische orale Dosierungsform gemäß Anspruch 13, dadurch gekennzeichnet, daß die Umhüllung eine Gelatinekapsel ist, die gegenüber Magensäften resistent gemacht worden ist.

16. Pharmazeutische orale Dosierungsform gemäß Anspruch 13, dadurch gekennzeichnet, daß die Gelatinekapselumhüllung gegenüber Magensäften mit Hilfe von Hydroxypropylmethylzellulosephthalat und Dibutylphthalat widerstandsfähig gemacht worden ist.

17. Pharmazeutische orale Dosierungsform gemäß Anspruch 13 oder 14, dadurch gekennzeichnet, daß die Magensaft-resistente und im Dünndarm zersetzbare Umhüllung eine Tablettenumhüllung ist, die gegenüber den Magensäften mit Hilfe von Zelluloseacetatphthalat widerstandsfähig gemacht worden ist.

18. Pharmazeutische orale Dosierungsform gemäß einem der vorhergehenden Ansprüche in Einheitsdosierungsform, enthaltend ungefähr 200 mg bis ungefähr 1500 mg des Bikarbonats.

19. Mittel zur Erhöhung des Plasmabikarbonatgehaltes eines Säugetiers, welches auch propyhlaktische oder therapeutische Wirkungen in dem Patienten bewirkt, in Form einer pharmazeutischen oralen Dosierungsform gemäß einem der vorhergehenden Ansprüche.

20. Mittel nach Anspruch 19, wobei die zu erzielende prophylaktische Wirkung die Verhinderung eines akuten Nierenversagens in einem Patienten ist, der gegenüber einem Nierenversagen anfällig ist.

21. Mittel nach Anspruch 19, wobei die zu erzielende prophylaktische Wirkung die Verhinderung der Bildung von Nierensteinen in einem Patienten, der gegenüber Nierensteinen anfällig ist, umfasst.

22. Mittel nach Anspruch 19, wobei die zu erzielende therapeutische Wirkung die Behandlung eines Säugetiers ist, das an einer abnormalen Retention von Körperflussigkeiten oder Retentionsprodukten leidet.

23. Mittel nach Anspruch 22, wobei die Behandlung einer abnormalen Verteilung oder Retention von Körperflüssigkeiten oder Retentionsprodukten die Behandlung eines Zustands einer metabolischen Azidose umfasst.

24. Mittel nach Anspruch 22, wobie die Behandlung der abnormalen Verteilung oder Retention von Körperflüssigkeiten die Behandlung eines Patienten umfasst, der an einer veränderten Nierenfunktion oder an einer Niereninsuffizienz leidet.

25. Mittel nach Anspruch 22, wobei die Behandlung der abnormalen Verteilung oder Retention von Körperflüssigkeiten die Behandlung eines Patienten umfasst, der an einer Flüssgkeitsüberlastung leidet.

26. Mittel nach Anspruch 22, wobei die Behandlung der abnormalen Verteilung oder Retention von Körperflüssigkeiten oder Retentionsprodukte die Behandlung eines Patienten umfasst, der an Nierensteinen leidet.

27. Behälter aus einer Vielzahl von Einheitsdosierungsformen gemäß Anspruch 18, der durch Indikationen über die Eignung seiner Inhalte für eine Verwendung bei der Prophylaxe oder Therapie einer abnormalen Verteilung oder Retention von Körperflüssigkeiten oder Retentionsprodukten in einem Säugetier begleitet ist.

28. Behälter aus einer Vielzahl von Einheitsdosierungsformen gemäß Anspruch 18, der von Instruktionen für ein Säugetier, eine spezifische Menge seines Inhalts für die Prophylaxe oder Therapie einer abnormalen Verteilung oder Retention von Körperflüssigkeiten oder Retentionsproduktion in Säugetieren zu schlucken.

29. Behälter nach Anspruch 28, dadurch gekennzeichnet, daß die in den Instruktionen angegebene spezifische Menge dazu ausreicht, einen pH-Wert des frischen Urins des Patienten im neutralen bis alkalischen Bereich bei einem pH von wenigstens 6 und vorzugsweise bei einem pH von zwischen 6,8 und 8 zu halten.

30. Behälter nach Anspruch 29, dadurch gekennzeichnet, daß die spezifische Menge, die in den Instruktionen angegeben ist, dazu ausreicht, den pH-Wert des frischen Urins aufrecht zu halten.

31. Behälter nach einem der Ansprüche 27 bis 30, dadurch gekennzeichnet, daß die Prophylaxe die Verhinderung eines akuten Nierenversagens in einem Patienten umfasst, der gegenüber einem Nierenversagen anfällig ist.

32. Behälter nach Anspruch 27 bis 30, dadurch gekennzeichnet, daß die Prophylaxe die Verhinderung der Bildung von Nierensteinen in einem Patienten, der gegenüber Nierensteinen anfällig ist, umfasst.

33. Behälter nach einem der Ansprüche 27 bis 30, dadurch gekennzeichnet, daß die Therapie einer abnormalen Verteilung oder Retention von Körperflüssigkeiten oder Retentionsprodukten die Behandlung eines Zustandes einer metabolischen Azidose umfasst.

34. Behälter nach einem der Ansprüche 27 bis 30, dadurch gekennzeichnet, daß die Therapie einer abnormale Verteilung oder Retention von Körperflüssigkeiten oder Retentionsprodukten die Behandlung eines Patienten umfasst, der an einer veränderten Nierenfunktion oder an einer Niereninsuffizienz leidet.

35. Behälter nach einem der Ansprüche 27 bis 30, dadurch gekennzeichnet, daß die Therapie einer abnormale Verteilung oder Retention von Körperflüssigkeiten oder Retentionsprodukten die Behandlung eines Patienten umfasst, der an einer Flüssigkeitsüberbelastung leidet.

36. Behälter nach einem der Ansprüche 27 bis 30, dadurch gekennzeichnet, daß die Therapie der abnormalen Verteilung oder Retention von Körperflüssigkeiten oder Retentionsprodukten die Behandlung eines Patienten umfasst, der an Nierensteinen leidet.

37. Verfahren zur Herstellung einer pharmazeutischen oralen Dosierungsform, die als Wirkstoff ein Bikarbonat als Bikarbonationen-bildende Substanz zur Bildung oder Freisetzung von resorbierbaren Bikarbonationen ohne Möglichkeit einer Reaktion derselben mit Magensäften im Magen des Patienten aufweist, dadurch gekennzeichnet, daß eine Lösung einer pH-empfindlichen Substanz, die bei einem pH von mehr als ungefähr 6 zersetzbar ist, hergestellt wird, die Substanz im wesentlichen frei von Wasser ist und das Lösungsmittel, das zur Herstellung der Lösung verwendet wird, in ähnlicher Weise im wesentlichen frei von Wasser ist, ein Kern aus dem Wirkstoff hergestellt wird, der ebenfalls im wesentlichen frei von Wasser ist, eine Amospha8re um den Kern herum erzeugt wird, die eine relative Feuchtigkeit von weniger als 40 % aufweist, die Lösung der pH-empfindlichen Substanz auf die Oberfläche des Kerns aufgebracht wird und das Lösungsmittel verdampfen gelassen wird zur Gewinnung einer pharmazeutischen oralen Dosierungsform des Wirkstoffs, der gleichmäßig und vollständig von dem Überzug aus der pH-empfindlichen Substanz umschlossen ist.

38. Verfahren nach Anspruch 37, dadurch gekennzeichnet, daß die pH-empfindlich Substanz aus Zellusloseacetatphthalat besteht.

39. Verfahren nach Anspruch 37 oder 38, dadurch gekennzeichnet, daß das Lösungsmittel, das für die Herstellung der Lösung der pH-empfindlichen Substanz verwendet wird, ein nicht-alkoholisches Kohlehydrat mit 1 bis 5 Kohlenstoffatomen ist.

40. Verfahren nach Anspruch 39, dadurch gekennzeichnet, daß das Lösungsmittel ein halogeniertes Kohlehydrat ist.

41. Verfahren nach Anspruch 40, dadurch gekennzeichnet, daß das Lösungsmittel aus Methylenchlorid besteht.

42. Verfahren nach einem der Ansprüche 37 bis 41, dadurch gekennzeichnet, daß ein oder mehr Erweichungsmittel, Pigmente und Schutzmittel zusätzlich der Lösung der pH-empfindlichen Substanz zugesetzt werden.

43. Verfahren nach einem der Ansprüche 37 bis 42, dadurch gekennzeichnet, daß die Menge der pH-empfindlichen Substanz in der Lösung oberhalb 5 Gew.-% liegt.

44. Verfahren nach Anspruch 43, dadurch gekennzeichnet, daß die Menge der pH-empfindlichen Substanz in der Lösung zwischen ungefähr 7 und 10 Gew.-% liegt.

45. Verfahren nach einem der Ansprüche 37 bis 44, dadurch gekennzeichnet, daß die Beschichtung der pH-empfindlichen Substanz mit einer relativen Geschwindigkeit in der Weise durchgeführt wird, daß ein relativ hoher Gewichtsanteil der pH-empfindlichen Substanz in der Lösung verwendet wird, ein leicht flüchtiges Lösungsmittel eingesetzt wird und die Atmosphäre um den Kern herum, auf welchen die Lösung aufgebracht wird, auf einer relativ hohen Temperatur von mehr als 35°C und vorzugsweise mehr als 40°C jedoch weniger als 55°C, gehalten wird.

46. Verfahren nach einem der Ansprüche 37 bis 45, dadurch gekennzeichnet, daß der Kern des Wirkstoffs in der Weise erzeugt wird, daß zuerst ein Granulat gebildet wird, das im wesentlichen frei von Wasser ist, das Granulat zur Gewinnung einer Kernform aus einer Einheitsdosierungsmenge des Wirkstoffs verpresst wird und der Kern von allen scharfen Ecken und Unregelmäßigkeiten an der Oberfläche zur Gewinnung einer polierten Oberflächenkernform befreit wird, die im wesentlichen frei von Wasser ist.

47. Verfahren nach Anspruch 46, dadurch gekennzeichnet, daß das Granulat den Wirkstoff, ein Granulierungsmittel, daß in einem Lösungsmittel aufgelöst ist, und gegebenenfalls ein Bindemittel enthält, wobei jede Komponente im wesentlichen frei von Wasser ist, und wobei das Granulat frei von Lösungsmittel ist, bevor es zur Gewinnung der Kernform verpresst wird.

48. Verfahren nach Anspruch 46 oder 47, dadurch gekennzeichnet, daß das Granulat mit einem Expandiermittel vermischt wird, um die Verteilung des Wirkstoffs zu begünstigen, wenn dieser im Darm eines Säugetiers freigesetzt wird.

49. Verfahren nach einem der Ansprüche 46 bis 48, dadurch gekennzeichnet, daß die Atmosphäre, welche die polierte Oberflächenkernform umgibt, im wesentlichen frei von allen Kernformkomponenten ist, bevor die Lösung der pH-empfindlichen Substanz auf die Oberfläche des Kerns aufgebracht wird.

50. Verfahren nach einem der Ansprüche 37 bis 49, dadurch gekennzeichnet, daß die Bikarbonationen-bildende Substanz aus Natriumbikarbonat besteht.

51. Verfahren nach einem der Ansprüche 37 bis 50, dadurch gekennzeichnet, daß das Bikarbonat mit einer Menge eines Karbonats vermischt wird.

52. Verfahren nach Anspruch 51, dadurch gekennzeichnet, daß das Karbonat aus Natriumkarbonat besteht.

53. Pharmazeutische orale Dosierungsform, die als Wirkstoff ein Bikarbonat als Bikarbonationen-bildende Substanz enthält, hergestellt nach dem Verfahren gemäß einem der Ansprüche 39 bis 52.

**Revendications**

1. Forme posologique pharmaceutique par voie orale, comprenant en tant que principe actif un bicarbonate servant de substance formant l'ion bicarbonate, le principe actif étant enfermé dans un moyen d'enveloppe tel que le principe actif ne soit exposé que dans l'intestin d'un sujet, pour former ou libérer des ions bicarbonate résorbables sans qu'ils puissent réagir avec les sécrétions gastriques dans l'estomac du sujet.

2. Forme posologique pharmaceutique par voie orale selon la revendication 1, dans laquelle la substance formant l'ion bicarbonate comprend le bicarbonate de sodium.

3. Forme posologique pharmaceutique par voie orale selon la revendication 1 ou la revendication 2, dans laquelle le bicarbonate est associé à une certaine quantité d'un carbonate.

4. Forme posologique pharmaceutique par voie orale selon la revendication 3, dans laquelle le carbonate est le carbonate de sodium.

5. Forme posologique pharmaceutique par voie orale selon l'une quelconque des revendications 1 à 4, dans laquelle le principe actif est associé à un principe actif supplémentaire convenant lui aussi à la prophylaxie et au traitement des calculs rénaux, choisi dans le groupe comprenant les sels de métaux alcalins ou de métaux alcalino-terreux de l'acide citrique, et l'urée.

6. Forme posologique pharmaceutique par voie orale selon l'une quelconque des revendications 1 à 5, dans laquelle le principe actif est associé à un composé du calcium convenant à la supplémentation du calcium chez le sujet.

7. Forme posologique pharmaceutique par voie orale selon la revendication 6, dans laquelle le composé du calcium est le gluconate de calcium.

8. Forme posologique pharmaceutique par voie orale selon l'une quelconque des revendications 1 à 7, dans laquelle le principe actif est associé à un composé du potassium convenant à la supplémentation du potassium chez le sujet.

9. Forme posologique pharmaceutique par voie orale selon la revendication 8, dans laquelle le composé du potassium est choisi dans le groupe comprenant le chlorure de potassium, le bicarbonate de potassium et le citrate de potassium.

10. Forme posologique pharmaceutique par voie orale selon l'une quelconque des revendications 1 à 9, dans laquelle des éléments en traces, reconnus comme contribuant au bien-être général des sujets, sont contenus dans la forme posologique par voie orale.

11. Forme posologique pharmaceutique par voie orale selon l'une quelconque des revendications 1 à 10, dans laquelle l'acide aspartique ou un de ses dérivés est contenu dans la forme posologique par voie orale.

12. Forme posologique pharmaceutique par voie orale selon la revendication 11, dans laquelle l'aspartate de magnésium ou le chlorhydrate de l'aspartate de magnésium sont contenus dans la forme posologique par voie orale.

13. Forme posologique pharmaceutique par voie orale selon l'une quelconque des revendications 1 à 12, dans laquelle le moyen d'enveloppe comprend un matériau résistant aux sécrétions gastriques et décomposable sous l'effet des sécrétions intestinales.

14. Forme posologique pharmaceutique par voie orale selon la revendication 13, dans laquelle le moyen d'enveloppe comprend une résine acrylique.

15. Forme posologique pharmaceutique par voie orale selon la revendication 13, dans laquelle le moyen d'enveloppe est une enveloppe du type capsule de gélatine, rendue résistante aux sécrétions gastriques.

16. Forme posologique pharmaceutique par voie orale selon la revendication 13, dans laquelle l'enveloppe du type capsule de gélatine est rendue résistante aux sécrétions gastriques à l'aide de phtalate d'hydroxypropyl-méthylcellulose et de phtalate de dibutyle.

17. Forme posologique pharmaceutique par voie orale selon la revendication 13 ou la revendication 14, dans laquelle l'enveloppe résistant aux sécrétions gastriques et décomposable dans l'intestin grêle est une enveloppe du type comprimé, rendue résistante aux sécrétions gastriques à l'aide d'acétophtalate de cellulose.

18. Forme posologique pharmaceutique par voie orale selon l'une quelconque des revendications 1 à 17, sous présentation unitaire, comprenant d'environ 200 mg à environ 1500 mg du bicarbonate.

19. En tant que principe augmentant le taux plasmatique du bicarbonate chez un sujet mammifère, et permettant aussi d'obtenir chez le sujet des effets prophylactiques ou thérapeutiques, forme posologique pharmaceutique par voie orale selon l'une quelconque des revendications 1 à 18.

20. Principe selon la revendication 19, dans lequel l'effet prophylactique à obtenir comprend la prévention d'une insuffisance rénale aiguë chez un sujet susceptible de contracter une insuffisance rénale.

21. Principe selon la revendication 19, dans lequel l'effet prophylactique à obtenir comprend la prévention de la formation de calculs rénaux chez un sujet ayant tendance à former des calculs rénaux.

22. Principe selon la revendication 19, dans lequel l'effet thérapeutique à obtenir consiste à traiter un sujet mammifère souffrant d'une rétention anormale de fluides corporels ou de produits de rétention.

23. Principe selon la revendication 22, dans lequel le traitement d'une répartition ou d'une rétention anormales de fluides corporels ou de produits de rétention comprend le traitement d'un état d'acidose métabolique.

24. Principe selon la revendication 22, dans lequel le traitement de la répartition ou de la rétention anormales des fluides corporels comprend le traitement d'un sujet souffrant d'une altération de la fonction rénale, ou d'une insuffisance rénale.

25. Principe selon la revendication 22, dans lequel le traitement de la distribution ou de la rétention anormales des fluides corporels comprend le traitement d'un sujet souffrant d'une surcharge de fluides.

26. Principe selon la revendication 22, dans lequel le traitement de la répartition ou de la rétention anormales des fluides corporels ou des produits de rétention comprend le traitement d'un sujet souffrant de calculs rénaux.

27. Conditionnement comprenant une pluralité de présentations unitaires selon la revendication 18, et étant accompagné d'indications concernant la convenance de son contenu à une utilisation dans la prophylaxie ou le traitement de la répartition ou de la rétention anormales de fluides corporels ou de produits de rétention chez un sujet mammifère.

28. Conditionnement comprenant une pluralité de présentations unitaires selon la revendication 18, et étant accompagné d'instructions selon lesquelles un sujet mammifère doit avaler une quantité spécifiée de son contenu pour la prophylaxie ou le traitement de la répartition ou de la rétention anormales de fluides corporels ou de produits de rétention chez un sujet mammifère.

29. Conditionnement selon la revendication 28, dans lequel la quantité spécifiée est indiquée dans les instructions comme étant suffisantes pour donner à l'urine fraiche du sujet un pH compris entre un pH neutre et un pH alcalin, pour un pH d'au moins 6,5 et de préférence un pH compris entre 6,8 et 8.

30. Conditionnement selon la revendication 29, dans lequel la quantité spécifiée est indiquée dans les instructions comme étant suffisantes pour maintenir ledit pH de l'urine fraiche.

31. Conditionnement selon l'une quelconque des revendications 27 à 30, dans lequel la prophylaxie comprend la prévention de l'insuffisance rénale aiguë chez un patient susceptible de contracter une insuffisance rénale.

32. Conditionnement selon l'une quelconque des revendications 27 à 30, dans lequel la prophylaxie comprend la prévention de la formation de calculs rénaux chez un sujet ayant tendance à former des calculs rénaux.

33. Conditionnement selon l'une quelconque des revendications 27 à 30, dans lequel le traitement d'une répartition ou d'une rétention anormales de fluides corporels ou de produits de rétention comprend le traitement d'un état d'acidose métabolique.

34. Conditionnement selon l'une quelconque des revendications 27 à 30, dans lequel le traitement d'une répartition ou d'une rétention anormales de fluides corporels ou de produits de rétention comprend le traitement d'un sujet souffrant d'une altération de la fonction rénale ou d'une insuffisance rénale.

35. Conditionnement selon l'une quelconque des revendications 27 à 30, dans lequel le traitement d'une répartition ou d'une rétention anormales de fluides corporels ou de produits de rétention comprend le traitement d'un sujet souffrant d'une surcharge de fluides.

36. Conditionnement selon l'une quelconque des revendications 27 à 30, dans lequel le traitement d'une répartition ou d'une rétention anormales de fluides corporels ou de produits de rétention comprend le traitement d'un sujet souffrant de calculs réanaux.

37. Procédé pour la production d'une forme posologique pharmaceutique par voie orale comprenant en tant que principe actif un bicarbonate servant de substance formant l'ion bicarbonate, pour former ou libérer des ions bicarbonate résorbables sans qu'ils aient la possibilité de réagir sur les sécrétions gastriques dans l'estomac du sujet, qui comprend les étapes consistant à préparer une solution d'une substance sensible au pH, qui est décomposable à un pH supérieur à environ 6, ladite substance étant presque complètement exempte d'eau et, de même, le solvant utilisé pour préparer ladite solution étant presque entièment exempt d'eau, à préparer un noyau dudit principe actif, qui est lui assui presque entièrement exempt d'eau, à créer autour dudit noyau une atmosphère ayant une humidité relative inférieure à 40%, à appliquer ladite solution de la substance sensible au pH sur la surface dudit noyau, et à permettre audit solvant de s'évaporer pour obtenir une forme posologique pharmaceutique par voie orale du principe actif, qui soit uniformément et complètement enfermée à l'intérieur d'un enrobage de ladite substance sensible au pH.

38. Procédé selon la revendication 37, dans lequel la substance sensible au pH est l'acétophtalate de cellulose.

39. Procédé selon les revendications 37 ou 38, dans lequel le solvant utilisé pour préparer la solution de la substance sensible au pH est un hydrocarbure non alcoolique contenant de 1 à 5 atomes de carbone.

40. Procédé selon la revendication 39, dans lequel le solvant est un hydrocarbure halogéné.

41. Procédé selon la revendication 40, dans lequel le solvant est le chlorure de méthylène.

42. Procédé selon l'une quelconque des revendications 37 à 41, dans lequel une ou plusieurs substances quelconques du groupe comprenant un agent adoucissant, un pigment et un agent protecteur, se trouvent de plus dans la solution de la substance sensible au pH.

43. Procédé selon l'une quelconque des revendications 37 à 42, dans lequel la quantité de la substance sensible au pH se trouvant dans la solution est supérieure à 5 % en poids.

44. Procédé selon la revendication 43, dans lequel la quantité de la substance sensible au pH se trouvant dans la solution est comprise entre environ 7 et 10 % en poids.

45. Procédé selon l'une quelconque des revendications 37 à 44, dans lequel l'application de la substance sensible au pH est effectuée à une vitesse relativement grande, par incorporation d'un pourcentage pondéral relativement élevé de la substance sensible au pH dans la solution, en utilisant un solvant facilement volatil et en maintenant la température autour du noyau sur lequel doit être appliquée la solution à une température relativement élevée, supérieure à 35°C et de préférence supérieure à 40°C, mais inférieure à 55°C.

46. Procédé selon l'une quelconque des revendications 37 à 45, dans lequel on produit le noyau du principe actif en fermant au départ un granulé presque entièrement exempt d'eau, en comprimant le granulé pour obtenir une forme du type noyau comprenant une quantité de doses unitaires du principe actif, et en supprimant du noyau tous les angles vifs et les irrégularités sur sa surface, pour obtenir un noyau à surface polie presque entièrement exempt d'eau.

47. Procédé selon la revendication 46, dans lequel ledit granulé comprend le principe actif, un agent de granulation dissous dans un solvant et, facultativement, un liant, chaque constituant étant presque entièrement exempt d'eau, et dans lequel ledit granulé est débarrassé du solvant avant d'être comprimé pour obtenir la forme noyau.

48. Procédé selon la revendication 46 ou la revendication 47, dans lequel ledit granulé est associé à un agent de dilatation, pour favoriser la répartition du principe actif quand il se libère dans l'intestin d'un sujet mammifère.

49. Procédé selon l'une quelconque des revendications 46 à 48, dans lequel l'atmosphère entourant la forme noyau à surface polie est entièrement débarrassée de tous les constituants de la forme noyau avant application de ladite solution de la substance sensible au pH sur la surface dudit noyau.

50. Procédé selon l'une quelconque des revendications 37 à 49, dans lequel la substance formant l'ion bicarbonate comprend du bicarbonate de sodium.

51. Procédé selon l'une quelconque des revendications 37 à 50, dans lequel le bicarbonate est associé à une certaine quantité d'un carbonate.

52. Procédé selon la revendication 51, dans lequel le carbonate est le carbonate de sodium.

53. Forme posologique pharmaceutique par voie orale comprenant en tant que principe actif un bicarbonate servant de substance formant l'ion bicarbonate, produite par le procédé selon l'une quelconque des revendications 39 à 52.